# EUROPÄISCHE PATENTANMELDUNG

(11) **EP 2 407 467 A1**
(43) Veröffentlichungstag der Anmeldung: **18.01.2012**
(21) Anmeldenummer: 10169520.3
(22) Anmeldetag: 14.07.2010
(51) Int. Cl.: C07D 403/12, A61K 31/551, A61P 19/10

(54) **Kristalline Verbindung von 1-[4-(2-azepan-1-yl-ethoxy)-benzyl]-2-(4-hydroxyphenyl)-3-methyl-1H-indol-5-ol und Milchsäure**

(71) Anmelder: SANDOZ AG, 4056 Basel (CH)
(72) Erfinder: Die Erfindernennung liegt noch nicht vor
(74) Vertreter: Pommerenke, Alexander

(57) **Zusammenfassung**

Die vorliegende Erfindung betrifft eine kristalline Verbindung von 1-[4-(2-azepan-1-yl-ethoxy)-benzyl] -2-(4-hydroxyphenyl)-3-methyl-1H-indol-5-ol (INN: Bazedoxifene) der Formel 1 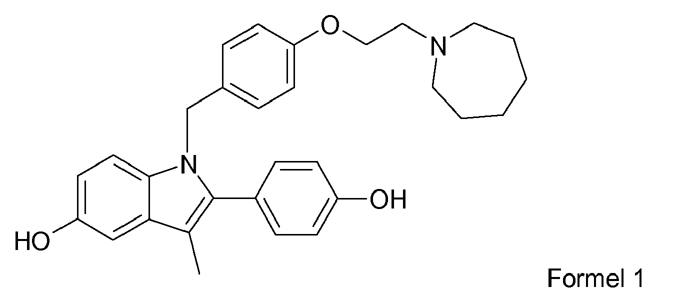 mit Milchsäure oder ein Hydrat hiervon, wobei das molare Verhältnis der Verbindung der Formel 1 zu Milchsäure 1:0,9 bis 1:1,1 ist, sowie ein Verfahren zu seiner Herstellung.

## Beschreibung

Die vorliegende Erfindung betrifft eine kristalline Verbindung von 1-[4-(2-azepan-1-yl-ethoxy)-benzyl] -2-(4-hydroxyphenyl)-3-methyl-1H-indol-5-ol (INN: Bazedoxifene) mit Milchsäure oder ein Hydrat hiervon, sowie ein Verfahren seiner Herstellung.

Bazedoxifen gehört zur Klasse der selektiven Östrogenrezeptor Modulatoren (SERMs). Selektive Östrogenrezeptormodulatoren (SERMs) werden als Substanzen definiert, die mit hoher Affinität an den Östrogenrezeptor binden und gleichzeitig keine signifikante Bindungsaktivität mit anderen nuklearen Rezeptoren besitzen. Im Gegensatz zu Östrogenen führen sie aber in den diversen Zielgeweben zu "östrogenagonistischer" oder "östrogenantagonistischer" Wirkung. Bazedoxifen ist wirksam in der Prävention und Therapie der Osteoporose und insbesondere der postmenopausalen Osteoporose. Eine detaillierte Beschreibung seiner Wirkung kann z.B. in Drugs of the Future, 2002, 27(2), 117-121 gefunden werden.

Die Herstellung von Bazedoxifen und seiner Acetate wird in US 5,998,402 und 6,479,535 offenbart. Die Herstellung von Bazedoxifen wurde auch in J.Med.Chem. 2001, 44, 1654-1657, beschrieben.

In der US 2005/0227965 A1 wird kristallines Bazedoxifenacetat offenbart. Insbesondere werden zwei polymorphe Formen Bazedoxifenacetat beschrieben. Allerdings enthält Bazedoxifenacetat gemäß der Lehre der US 2005/0227965 A1 Verunreinigungen und ist instabil.

Gemäss dem Stand der Technik ist die Form B Bazedoxifenacetat die thermodynamisch stabile Form während die Form A kinetisch stabil ist. Aus diesem Grund sind die Bedingungen zur Darstellung reiner Form A oder reiner Form B kritisch, insbesondere wenn das zur Kristallisation verwendete Lösungsmittel identisch ist, wie es in der US 7,683,051 und US 7,683,053 beschrieben wird.

Die W02009/012734 A2 offenbart Salze von Bazedoxifen mit Polycarbonsäuren. Die ungewünschte Zersetzung des Bazedoxifenacetats soll vermieden werden, indem neue Salze des Bazedoxifens zur Verfügung gestellt werden. In der W02009/012734 A2 und US 2005/0227965 A1 werden keine kristallinen Verbindungen enthaltend Bazedoxifen und Milchsäure offenbart.

Die Verwendung einer Substanz als Arzneimittel bedingt eine hohe Substanzqualität der pharmazeutisch aktiven Substanz. Eines der effektivsten Reinigungsverfahren ist die Kristallisation. Falls eine Substanz nicht kristallisiert kann es schwierig sein die international anerkannten Qualitätskriterien der ICH (International Commission for Harmonization) einzuhalten. Des Weiteren neigen amorphe Substanzen oftmals zu einer leichteren Zersetzung, insbesondere zur Hydrolyse oder Oxidation, was durch die höhere freie Energie und durch die typischerweise grössere Oberfläche bedingt wird. Zusammenfassend lässt sich feststellen, dass Bazedoxifen und die bis jetzt bekannten Formen hiervon schwierig zu Reinigen sind und zur Zersetzung neigen.

Die technische Aufgabe der vorliegenden Erfindung ist die Bereitstellung kristalliner Verbindungen enthaltend Bazedoxifen, die ein vorteilhaftes Eigenschaftsprofil aufweisen. Insbesondere sollen diese Verbindungen leicht erhältlich sein, eine gute Stabilität aufweisen und frei von Zersetzungsprodukten sein. Des Weiteren soll ein Verfahren zur Bereitstellung der vorgenannten Verbindungen zur Verfügung gestellt werden.

Die technische Aufgabe der vorliegenden Erfindung wird durch eine kristalline Verbindung von 1-[4-(2-azepan-1-yl-ethoxy)-benzyl] -2-(4-hydroxyphenyl)-3-methyl-1H-indol-5-ol (INN: Bazedoxifene) der Formel 1 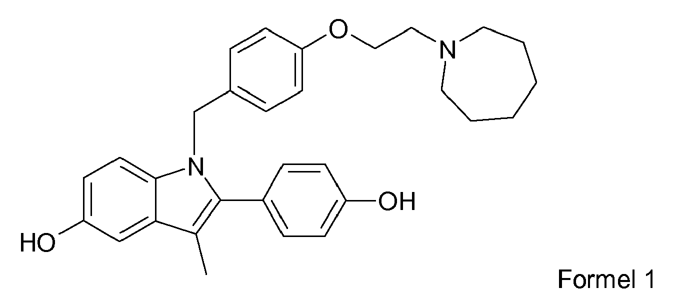 mit Milchsäure oder ein Hydrat hiervon, wobei das molare Verhältnis der Verbindung der Formel 1 zu der Säure 1:0,9 bis 1:1,1 ist, gelöst.

Überraschenderweise sind die vorgenannten Verbindungen leicht erhältlich und weisen eine gute Stabilität auf.

Überraschenderweise bildet Milchsäure und Bazedoxifen eine kristalline Verbindung mit dem oben angegebenen molaren Verhältnis. In einer bevorzugten Ausführungsform ist das molare Verhältnis der Verbindung der Formel 1 und Milchsäure 1:0,95 bis 1:1,05 und am meisten bevorzugt 1:1 ist

Amorphe Formen sind in der Regel dadurch gekennzeichnet, dass diese keine scharfen Pulverdiffraktions-Reflexe zeigen. Das Pulverdiagramm von amorphen Formen besitzt lediglich ein stark erhöhtes Untergrundsignal im º2θ Bereich von ca. 10° bis 30°. Mesomorphe Formen sind normalerweise dadurch gekennzeichnet, dass diese nur sehr wenige, oft nur ein oder zwei, oder gar keine scharfen Pulverdiffraktions-Reflexe aufweisen. Mischungen von amorphen und kristallinen Phasen weisen vor allen dann nur sehr schwache Pulverdiffraktions-Reflexe auf, wenn der amorphe Anteil sehr hoch ist; zum Beispiel 50% oder höher.

Röntgen-Pulverdiffraktion ist eine weit verbreitete und anerkannte Methode zu Identifizierung und Charakterisierung von molekularen Festkörpern. Beschreibungen dieser Methode findet man sowohl in der Europäischen Pharmakopöe, als auch in der US Pharmacopeia als Methode Nr. 941 "X-Ray Diffraction", oder in "Polymorphism - In the Pharmaceutical Industry" Kapitel 6, Rolf Hilfiker, Editor, Wiley-VCH Verlag, Weinheim, Deutschland, 2006. Normalerweise wird Röngten-Pulverdiffraktion mit Kupfer-K_{α} Strahlung durchgeführt, wie dies auch hier der Fall war. D-Werte in 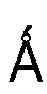 (d) und 2-theta (2θ) Werte in Winkelgrad lassen sich durch die Bragg'sche Gleichung wie folgt ineinander umrechnen: nλ = 2dsinθ, wobei n eine ganze Zahl und λ die Wellenlänge der verwendeten Röntgenstrahlung in A ist. Die aus einer Pulverdiffraktionsmessung erhalten Daten sind Signalintensität (in Counts) in Abhängigkeit des Winkels 2θ als Messgrösse. Im Weiteren ist zu beachten, dass sowohl in Reflexionsgeometrie, als auch in Transmissionsgeometrie gemessen werden kann. Die hier erwähnten Messungen wurden in Reflexionsgeometrie ausgeführt. Während die Lage der Linien in Winkelgrad nicht von der Geometrie abhängig ist, können sich aber die relativen Intensitäten sowohl durch die Geometrie, als auch durch die Eigenschaften der Probe und durch die Probenpräparation ändern. Deshalb dienen die angegeben Intensitäten nur als qualitatives Merkmal. Bei Messungen dieser Art beträgt der Messfehler üblicherweise ±0,2° 2θ. Während der Messfehler für °2θ sich über den gesamten Messbereich nur geringfügig oder gar nicht verändert, ist aufgrund der oben erwähnten Bragg'schen Gleichung der Fehler bei den d-Werten abhängig vom Winkel. 2θ Winkel und d-Werte sind aber äquivalent und deshalb ist für die d-Werte kein Messfehler ausgerechnet worden, obwohl ein solcher vorliegt.

Eine Besonderheit der hier vorliegenden Erfindung sind kristalline Verbindungen von Bazedoxifen, welche sich durch eine hohe kristalline Reinheit hervorheben. Die kristallinen Verbindungen der vorliegenden Erfindung besitzen scharfe Pulverdiffraktions-Reflexe und enthalten nur geringe amorphe Anteile.

Raman Spektroskopie ist eine zweite sehr nützliche Methode zur Identifizierung und Charakterisierung verschiedener Formen von molekularen Festkörpern. Detailliertere Beschreibungen der Anwendung von Raman Spektroskopie zum erwähnten Zweck findet man z. B. in "Polymorphism - In the Pharmaceutical Industry" Kapitel 5, Rolf Hilfiker, Editor, Wiley-VCH Verlag, Weinheim, Deutschland, 2006. Bei Messungen dieser Art beträgt der Messfehler üblicherweise ±1 cm⁻¹_{.}

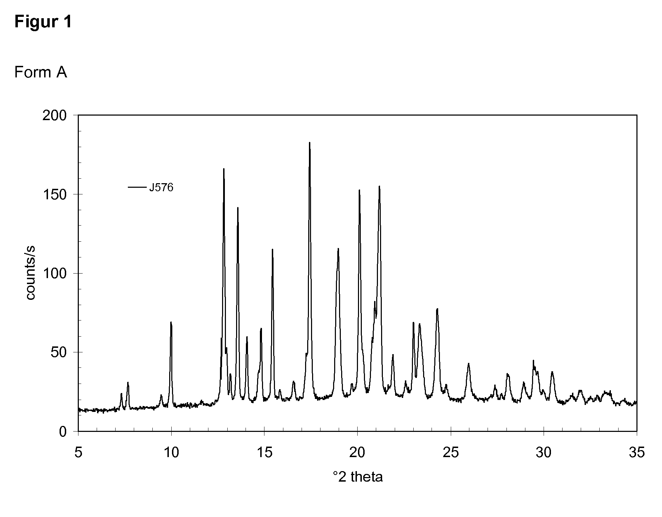

Vorzugsweise weist ist die Verbindung ein Röntgenpulverdiffraktogramm (XRPD) mit wenigstens einem Reflex (ausgedrückt als 2θ ± 0,2° 2θ (Cu_{Ka} Strahlung)) bei 12.8°, 13.6°, 17.4°, 20.1 ° und 21.2° auf, die nachfolgend als Form A bezeichnet wird. Vorzugsweise weist Form A wenigstens einen Reflex 2θ bei 10.0°, 12.8°, 13.6°, 15.5°, 17.4°, 19.0°, 20.1°, 21.2° und 24.3° auf. Figur 1 zeigt ein Röntgenpulverdiffraktogramm der Form A.

In einer weiter bevorzugten Ausführungsform enthält Form A L-Milchsäure, D-Milchsäure oder razemische Milchsäure, wobei L-Milchsäure besonders bevorzugt ist.

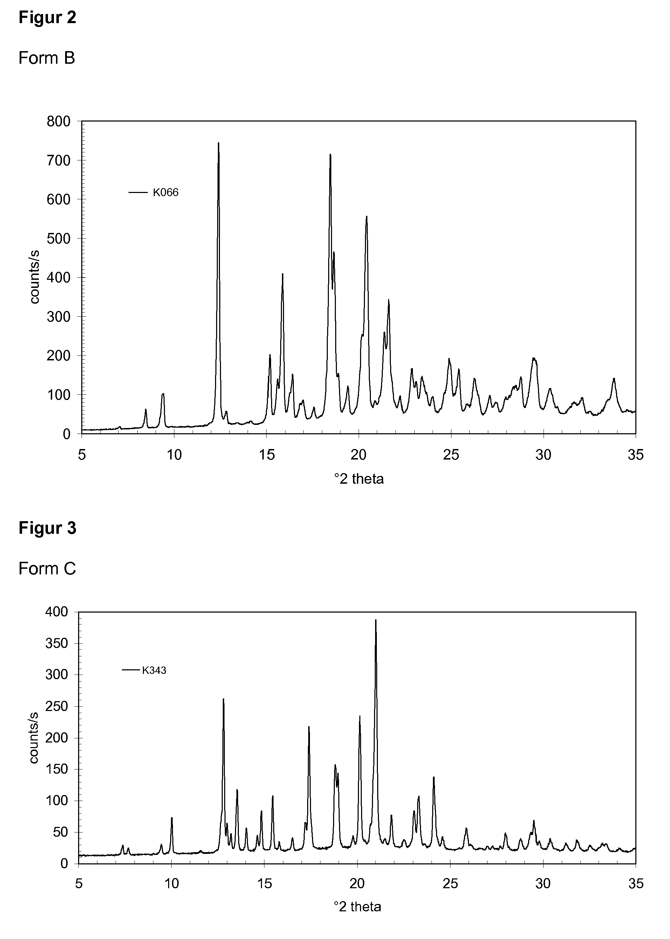

Weiter bevorzugt weist die Verbindung ein Röntgenpulverdiffraktogramm (XRPD) mit wenigstens einem Reflex (ausgedrückt als 2θ ± 0,2° 2θ (Cu_{Kα} Strahlung)) bei 12.4°, 15.9°, 18.5° und 20.4° auf, die nachfolgend als Form B bezeichnet wird. Vorzugsweise weist Form B wenigstens einen Reflex 2θ bei 9.4°, 12.4°, 15.2°, 15.9°, 18.5°, 18.7°, 20.4°, 21.4° und 29.5° auf. Figur 2 zeigt ein Röntgenpulverdiffraktogramm der Form B.

Im Weiteren weist die Form B vorzugsweise ein charakteristisches Ramanspektrum mit Spektralbanden bei 3056, 1612, 1568, 1471, 1422, 1358, 1276, 1173, 1087, 923, 843, 820, 798, 707, 634, 417, 352 and 286 cm⁻¹ (Wellenzahlen) auf.

In einer weiter bevorzugten Ausführungsform enthält Form B L-Milchsäure, D-Milchsäure oder razemische Milchsäure, wobei L-Milchsäure besonders bevorzugt ist.

Weiter bevorzugt weist die Verbindung ein Röntgenpulverdiffraktogramm (XRPD) mit wenigstens einem Reflex (ausgedrückt als 2θ ± 0,2° 2θ (Cu_{Ka} Strahlung)) bei 21.0°, 23.3° und 24.1 ° auf, die nachfolgend als Form C bezeichnet wird. Vorzugsweise weist Form C wenigstens einen Reflex 20 bei 10.0°, 12.8°, 15.5°, 17.4°, 18.8°, 21.0°, 23.3° und 24.1 auf. Figur 3 zeigt ein Röntgenpulverdiffraktogramm der Form C. In einer weiter bevorzugten Ausführungsform enthält Form C L-Milchsäure, D-Milchsäure oder razemische Milchsäure, wobei L-Milchsäure besonders bevorzugt ist.

Die vorgenannten kristallinen Verbindungen enthaltend Milchsäure und insbesondere die Formen A, B und C sind nicht hygroskopisch. Deshalb weisen sie eine verbesserte Stabilität auf, wenn sie in Umbebungen mit einer hohen Luftfeuchte gelagert werden. Dies ist besonders vorteilhaft, wenn Medikamente enthaltend die kristalline Verbindung in tropischen und sub-tropischen Gegenden verabreicht werden, da die Medikamente somit eine gute Lagerstabilität aufweisen.

Des Weiteren können die Formen A, B und C mit einer guten Polymorphenreinheit von vorzugsweise mehr als 90 %, weiter bevorzugt mehr als 95% und insbesondere von mehr als 99% erhalten werden.

Ein weiterer Vorteil ist, daß die Formen A, B und C durch Kristallisation aus geeigneten Lösungsmitteln in hoher Reinheit erhältlich sind. Form A kann beispielsweise in hoher Reinheit durch Kristallisation aus Ethanol erhalten werden. Form B vorzugsweise durch Kristallisation aus Ethylacetat. Form C ist leicht durch Kristallisation aus Isopropanol erhältlich.

Ein weiterer Gegenstand der vorliegenden Erfindung ist vorgenannte kristalline Verbindung zur Anwendung in der Behandlung von Osteoporose, insbesondere von postmenopausaler Osteoporose. Ein Medikament enthaltend die vorgenannte kristalline Verbindung ist ein weiterer Gegenstand der vorliegenden Erfindung.

Des Weiteren kann die vorgenannte kristalline Verbindung zur Herstellung eines Medikamentes zur Behandlung von Osteoporose, insbesondere von postmenopausaler Osteoporose verwendet werden.

Die Formulierungen des Medikamentes beinhalten einen wirksamen Anteil an kristalliner Verbindung der mit einer Tagesdosis von 0,1 mg bis 200 mg verabreicht wird. Eine solche Dosis kann mittels jeder Darreichungsform verabreicht werden, die den Eintritt der wirksamen Verbindung in den Blutstrom ermöglicht, einschliesslich oraler Gabe, mittels Implantaten, parenteraler Gabe (einschliesslich intravenöser, intraperitonealer und subkutaner Injektion) and transdermaler Gabe.

Orale Formulierungen enthaltend die kristalline Verbindung der vorliegenden Erfindung beinhalten jegliche üblicherweise verwendete orale Darreichungsform wie Tabletten, Kapseln, Säfte, Suspensionen und Lösungen. Kapseln oder Tabletten können mit Mischungen anderer pharmazeutisch aktiver Substanzen, inerten Füllstoffen oder Verdünnungsmitteln wie pharmazeutisch akzeptablen Stärken, Zuckern, künstlichen Süssstoffen, pulverförmiger Zellulose, Gelatine, Pflanzengummi oder Ähnlichem hergestellt werden.

Tabletten enthaltend die kristalline Verbindung der vorliegenden Erfindung können durch konventionelles Verpressen, Nassgranulation, Trockengranulation oder unter Einsatz von pharmakologisch akzeptablen Verdünnungsmitteln (Füllstoffen), Bindemitteln, Schmiermitteln, Desintegrationsmitteln, Suspendierungs- oder Stabilisierungsmitteln wie beispielsweise Magnesiumstearat, Stearinsäure, Talkum, Natriumlaurylsufat, mikrokristalline Zellulose, Calciumarboxymethylcellulose, Polyvinylpyrollidone, Gelatine, Alininsäure, Gummiarabikum, Xanthan, Natriumcitrat, komplexe Silicate, Kalziumcarbonat, Glycin, Dextrin, Sucrose, Sorbitol, Dicalciumphosphat, Calciumsulfat, Lactose, Kaolin, Mannitol, Natriumchlorid und/oder gepulvertem Zucker erhalten werden.

Orale Formulierungen können eine konventionelle Abgabe oder eine verzögerte Abgabe an Wirkstoff bedingen. Beispiele von Arzneiträgerstoffen zur Herstellung von Formulierungen enthaltend die kristalline Verbindung der vorliegenden Erfindung schliessen einen oder mehrere Füllstoffe, Desintegrationsmittel und Gleitmittel ein. Bevorzugte Beispiele von Formulierungen enthaltend die kristalline Verbindung der vorliegenden Erfindung werden in der US 2007/0048347 offenbart.

Ein weiterer Gegenstand der vorliegenden Erfindung ist ein Verfahren zur Herstellung der vorgenannten Verbindung umfassend die Schritte:
a) Bereitstellung einer Lösung von 1-[4-(2-azepan-1-yl-ethoxy) -benzyl] -2-(4-hydroxyphenyl)-3-methyl-1H-indol-5-ol (INN: Bazedoxifene) der Formel 1 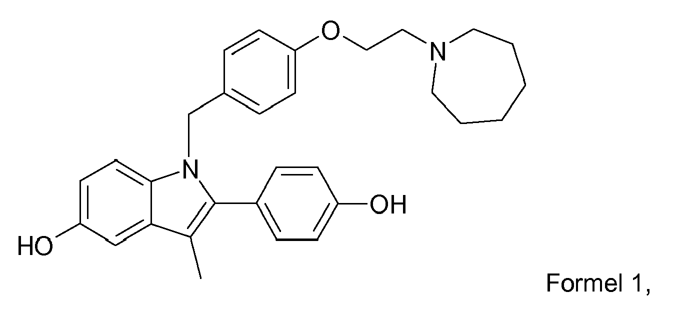
b) Zugabe von Milchsäure zu der Lösung des Schrittes a);
c) optionales Aufkonzentrieren der nach Schritt b) erhaltenen Zusammensetzung und/oder optionale Zugabe eines geeigneten Nichtlösungsmittels zur Erniedrigung der Löslichkeit der kristallinen Verbindung und
d) Abtrennung des erhaltenen Feststoffes.

Vorzugsweise ist das molare Verhältnis der gelösten Verbindung der Formel 1 in Schritt a) zu Milchsäure in Schritt b) 1:0,9 bis 1:1,1.

In einer weiter bevorzugten Ausführungsform wird die Säure in Schritt b) in Substanz zu der Lösung des Schrittes a) gegeben oder die Säure des Schrittes b) wird als eine Lösung in einem geeigneten Lösungsmittel zu der Lösung des Schrittes a) gegeben.

Das Lösungsmittel des Schrittes a) und/oder b) ist vorzugsweise ein niedermolekulares und physiologisch verträgliches Lösungsmittel und/oder ein physiologisch verträglicher Alkohol. wie z. B. Ethanol, Isopropanol, 1-Propanol, n-Butanol, niedermolekulare Ketone, wie z. B. Aceton, 2-Butanon, Methyl-Isobutyl Keton, Acetate, z. B. Ethyl Formiat, Ethyl Acetate, Butyl Acetat oder Isopropyl Acetat, oder Ether, z. B. tert-Butyl Methyl Ether, Diäthyläther, Diisopropyläther oder beliebig zu kombinierende Mischungen hiervon eingesetzt. Besonders bevorzugt für den Schritt a) und/oder Schritt b) sind physiologisch verträgliche Lösungsmittel in welchen die Milchsäure in genügender Weise löslich ist. Bevorzugt sind Ethanol, 1-Propanol, 2-Propanol, THF, Aceton, Ethylmethyl Keton, Wasser und Mischungen derselben. Besonders bevorzugt für Schritt a) sind Ethanol, 2-Propanol, Ethyl Acetat, Isopropyl Acetat, Aceton, Ethylmethyl Keton und für Schritt b) Ethanol, 2-Propanol, Aceton, Ethylmethyl Keton und Wasser.

In den Schritten a) und b) können die beiden Komponenten sowohl in gleichen als auch in unterschiedlichen Lösungsmitteln, und sowohl in gleichen als auch in unterschiedlichen Konzentrationen gelöst werden. Es ist auch möglich, jeweils Lösungsmittelgemische aus zwei oder mehr Lösungsmitteln in dem Schritt a) und/oder Schritt b) einzusetzen.

In Schritt c) können vorzugsweise Impfkristalle der gewünschten Form zugegeben werden. In Schritt c) ist es weiter bevorzugt, dass die erhaltene Suspension bei einer geeigneten Temperatur gerührt wird. Vorzugsweise liegt diese Temperatur im Bereich von 5 °C bis 50 °C.

Weiter kann in den Schritten a) und b) die Kristallisationstemperatur, respektive das Erreichen der Sättigung bezüglich des gewünschten Salzes durch die Auswahl verschiedener Kombinationen der Lösungsmittel und Konzentrationen so moduliert werden, dass das gewünschte Salz in hoher Ausbeute und hoher Reinheit erhalten wird.

Mit physiologisch verträglich ist gemeint, dass diese Lösungsmittel unter die ICH (International Commission for Harmonization) Richtline Q3C, Klasse 3, fallen. In der Reihenfolge können die Schritte a) und b) vertauscht werden.

In einer weiter bevorzugten Ausführungsform ist die Säure L-Milchsäure, D-Milchsäure oder razemische Milchsäure, wobei L-Milchsäure besonders bevorzugt ist.

Vorzugsweise wird die Verbindung der Formel 1 in Schritt a) in einer Lösung enthaltend Ethanol, Ethylacetat und/oder Isopropanol vorgelegt. Es ist weiter bevorzugt, dass in Schritt b) Ethanol und/oder Isopropanol als Lösungsmittel eingesetzt wird. Weiter bevorzugt wird in Schritt b) eine Lösung enthaltend Milchsäure und Ethanol, Ethylacetat und/oder Isopropanol als Lösungsmittel zugegeben. Vorzugsweise erhält man so die Form A, B oder C.

Unter Verwendung von Ethanol als Lösungsmittel erhält man vorzugsweise die Form A. Die Verwendung von Ethylacetat als Lösungsmittel bedingt vorzugsweise die Bildung von Form B. Isopropanol als Lösungsmittel führt vorzugsweise zur Form C.

### Beispiele

### Röntgen-Pulverdiffraktion

Pulverdiffraktogramme wurden auf einem Bruker D8 Advance Pulverdiffraktometer mit einem Goniometerradius von 217.5 mm unter Verwendung von Kupfer Kα Strahlung gemessen. Dieses Gerät arbeitet in Reflektions Bragg-Brentano Geometrie bei einer Anodenspannung von 40 kV und einem Strom von 40 mA, wobei eine variable Divergenzblende verwendet wurde. Das Bruker D8 Gerät ist ausgerüstet mit einem LynxEye Detektor, wobei das aktive Beobachtungsfenster auf 3° eingestellt wurde. Die Schrittweite betrug 0.02° und die äquivalente Akkumulationszeit 37 Sekunden pro Schritt. Die Proben wurden ohne weitere Behandlung auf zirkulären Silizium-Einkristall Trägern mit einer Tiefe von 0.1 mm und einem Durchmesser von 12 mm präpariert. Die Proben wurden während der Messung mit 0.5 Drehungen pro Sekunde rotiert. Der Messfehler beträgt etwa ±0,1 ° 26.

### Raman Spektroskopie

Fourier Transformation Raman Spektroskopie wurde mit einem Bruker RFS100 durchgeführt, welches zur Anregung einen Nd:YAG Laser mit einer Wellenlänge von 1064 nm besitzt. Die verwendete Laserleistung war 300 mW. Das verwendete Gerät ist mit einem Flüssig-Stickstoff gekühlten Germanium Detektor ausgerüstet. Ca. 3 mg der Messprobe werden in einen kleinen Aluminiumträger gepresst und dieser Träger wird in die Spektrometer-Messkammer eingesetzt. Zur Aufnahme von Spektren wurden 64 Akkumulationen mit einer Auflösung von 2.0 cm⁻¹ im Bereich von 100-3500 cm⁻¹ Wellenzahlen gemessen. Der Messfehler beträgt etwa ±1 cm⁻¹.

### Wasserdampfadsorptionsmessungen

Dynamische Wasserdampfadsorptionsmessungen (DVS) wurden mit einem SPS11-100n Gerät, hergestellt von der Firma "Projekt Messtechnik" in Ulm, Deutschland durchgeführt. Dazu wurden ca. 20 mg der Probe in einen Aluminiumträger eingewogen und dieser in der Messkammer des Geräts eingesetzt. Die Probe wurde dann gemäss einem definierten Programm vorgewählten relativen Feuchten ausgesetzt, wobei die Massenänderung über die Zeit bestimmt wird. Das folgende Mess-Programm wurde verwendet: 50% r.h. konstant während zwei Stunden, dann Änderung der relativen Feuchte auf 0% r.h. anschliessend Änderung der relativen Feuchte auf 96% r.h., gleichbleibend 96% r.h. während vier Stunden, und dann Änderung der relativen Feuchte auf 50% r.h. und dann gleichbleibend 50% r.h. während einer Stunde. Die eingestellten Änderungsraten waren jeweils 5% pro Stunde.
¹H-NMR Spektroskopie
¹H-NMR Spektroskopie wurde auf einem Bruker DPX300 Gerät durchgeführt.

### Beispiel 1: Herstellung der kristallinen Verbindung enthaltend Bazedoxifen und L-Milchsäure (Form A)

279 mg amorphes Bazedoxifenacetat werden in 4,0 ml Ethanol gelöst und vorgelegt. 10 ml einer 1M Stammlösung von L-Milchsäure werden hergestellt indem Ethanol zu 906 mg L-Milchsäure bis zu einem Gesamtvolumen von 10 ml gegeben werden. 1,0 ml dieser Stammlösung werden zu der Lösung enthaltend amorphes Bazedoxifenacetat gegeben. Die erhaltene Lösung wird unter Stickstoff eingeengt und nach vollständiger Entfernung des Lösungsmittels wird der Rückstand in Ethanol aufgenommen und erneut eingeengt. Nachfolgend wird der Rückstand mit 3,0 ml Ethanol aufgenommen und die erhaltene Lösung bei Raumtemperatur (22±2 °C) gerührt. Nach 20 h rühren wird eine Suspension erhalten. Das so erhaltene kristalline Produkt wird abfiltriert und an der Luft getrocknet. Laut ¹H-NMR wurde das Bazedoxifen L-Lactat Salz erhalten, welches nachfolgend als Form A bezeichnet wird. Das Röntgenpulverdiagramm ist in Figur 1 wiedergegeben. Es zeigt die in Tabelle 1 angegebenen Reflexe.

**Tabelle 1: Reflexe der Form A in °2θ und d-Werten**

| Pos. [°2θ.] | d-Werte [Å] | Qualitative Intensität |
|---|---|---|
| 7.3 | 12.1 | w |
| 7.7 | 11.5 | w |
| 9.5 | 9.3 | w |
| 10.0 | 8.9 | m |
| 12.8 | 7.0 | vs |
| 13.2 | 6.8 | w |
| 13.6 | 6.7 | s |
| 14.1 | 6.5 | m |
| 14.8 | 6.0 | m |
| 15.5 | 5.72 | s |
| 16.6 | 5.35 | w |
| 17.4 | 5.08 | vs |
| 19.0 | 4.67 | s |
| 20.1 | 4.40 | vs |
| 21.2 | 4.20 | vs |
| 21.9 | 4.08 | w |
| 23.0 | 3.86 | m |
| 23.4 | 3.81 | m |
| 24.3 | 3.66 | m |
| 26.0 | 3.42 | w |
| 28.1 | 3.18 | w |
| 29.0 | 3.08 | w |
| 29.5 | 3.02 | w |
| 30.5 | 2.93 | w |

### Beispiel 2: Herstellung der kristallinen Verbindung enthaltend Bazedoxifen und Milchsäure (Form B)

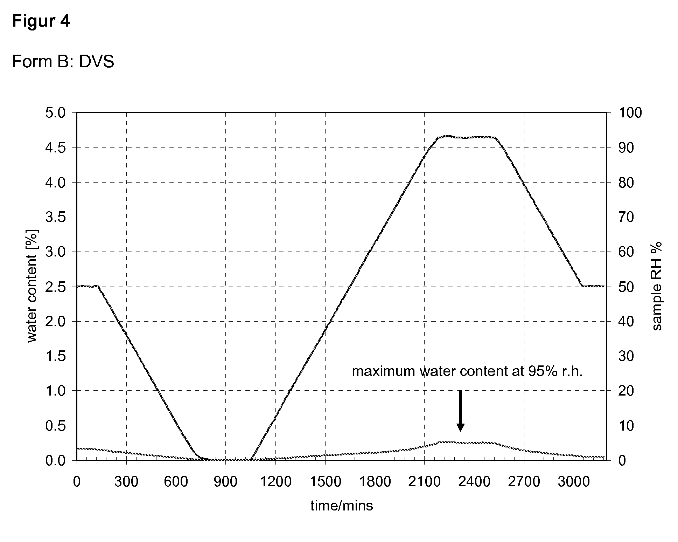

Zu 1,06 g Bazedoxifenacetat werden 20 ml Wasser und ein Äquivalent NaOH in Form einer 1M wässrigen Lösung gegeben. Der pH-Wert wird durch weitere Zugabe der 1M NaOH Lösung auf pH 11 eingestellt und die wässrige Phase sechs mal mit 30 ml CH₂Cl₂ extrahiert. Die ersten drei organischen Fraktionen werden vereinigt, filtriert und bis zur Trockne im Rotationsverdampfer eingeengt. Der Rückstand von etwa 400 mg bis 500 mg wird in 15 ml Ethylacetat aufgenommen. Zu der so erhaltenen Lösung wird sofort 2,0 ml der 1M L-Milchsäure Stammlösung (Beispiel 1) gegeben. Die erhaltene Lösung wird unter Stickstoff eingeengt, nach Trocknung in 3,0 ml Ethylacetat aufgenommen und bei Raumtemperatur gerührt. Etwa 10 mg Impfkristalle der Form A aus Beispiel 1 werden zugegeben und die Suspension wird kurz in einem Ultraschallbad mit Ultraschall beaufschlagt. Die erhaltene Suspension wird für drei Tage bei Raumtemperatur gerührt und eine Suspension des Bazedoxifenlactates wird erhalten. Das feste Produkt wird mit einem 0,45 µm Filter abgetrennt und bei Raumtemperatur getrocknet. Laut ¹H-NMR Spektroskopie weist das feste Produkt eine Stöchiometrie von Bazedoxifen zu Milchsäure von etwa 1:1 auf, welches als Form B bezeichnet wird. Das Röntgenpulverdiagramm (Form B) ist in Figur 2 wiedergegeben. Es zeigt die in Tabelle 2 angegebenen Reflexe. Die erhaltenen Kristalle der Form B werden einer Wasserdampfadsorptionsmessung unterzogen und man erhält das in Figur 4 angegebene Diagramm. Die Wasseraufnahme des erhaltenen Feststoffes ist weniger als 0,3 % bei einer relativen Feuchte von 95%.

**Tabelle 2: Reflexe der Form B in °2θ und d-Werten**

| Pos. [°2θ.] | d-Werte [Å] | Qualitative Intensität |
|---|---|---|
| 7.0 | 12.6 | VW |
| 8.4 | 10.5 | w |
| 9.4 | 9.4 | w |
| 12.4 | 7.1 | vs |
| 12.8 | 6.9 | VW |
| 15.2 | 5.85 | m |
| 15.6 | 5.69 | w |
| 15.9 | 5.60 | S |
| 16.4 | 5.41 | w |
| 16.9 | 5.23 | w |
| 18.5 | 4.81 | vs |
| 18.7 | 4.76 | s |
| 18.9 | 4.70 | w |
| 20.1 | 4.41 | m |
| 20.4 | 4.35 | vs |
| 21.4 | 4.16 | m |
| 21.6 | 4.11 | s |
| 22.8 | 3.89 | m |
| 23.1 | 3.85 | w |
| 23.4 | 3.80 | w |
| 24.9 | 3.58 | m |
| 25.4 | 3.51 | w |
| 26.2 | 3.39 | w |

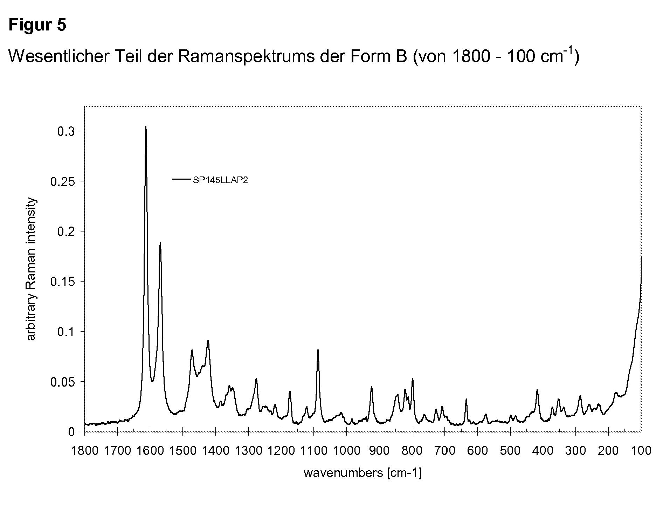

Die Form B weist ein Ramanspektrum wie in Figur 5 angegeben auf. Es zeigt die in Tabelle 3 aufgeführten Banden.

**Tabelle 3: Raman Spektralbanden der Form B**

| Wellenzahl [cm-¹] | relative Intensität in % (auf ganze Zahlen gerundet) |
|---|---|
| 3056 | 5 |
| 1612 | 31 |
| 1568 | 14 |
| 1471 | 3 |
| 1422 | 8 |
| 1358 | 3 |
| 1276 | 4 |
| 1173 | 3 |
| 1087 | 8 |
| 923 | 4 |
| 843 | 2 |
| 820 | 2 |
| 798 | 5 |
| 707 | 2 |
| 634 | 3 |
| 417 | 3 |
| 352 | 2 |
| 286 | 2 |

### Beispiel 3: Herstellung der kristallinen Verbindung enthaltend Bazedoxifen und Milchsäure (Form B)

Zu einer Lösung von 2,60 g Bazedoxifen in Form seiner freien Base in 20 ml Ethanol werden 6,0 ml der 1M L-Milchsäure Stammlösung (Beispiel 1) in Ethanol gegeben. 30 mg von Bazedoxifen L-Laktat aus Beispiel 2 werden zugegeben und ein schwacher Stickstoffstrom von etwa 20 ml/min wird über die Lösung in einer Glasflasche geleitet, bis die Hälfte des Lösungsmittels entfernt ist. 20 ml Heptan werden zugegeben und die erhaltene Suspension wird über Nacht bei Raumtemperatur gerührt. Der erhaltene Feststoff wird abfiltriert und im Vakuum für 3,5 Stunden bei 40 °C getrocknet. Das feste Produkt zeigt ein Röntgenpulverdiagramm wie in Figur 2 angegeben. Laut ¹H-NMR ist in dem erhaltenen Feststoff das Verhältnis von Bazedoxifen zu L-Milchsäure 1:1. Eine thermogravimetrische Analyse zeigt einen Gewichtsverlust des Produktes von 0,12%, wenn es mit 10 K/min auf 125 °C erhitzt wird. Dies bestätigt die sehr guten Trocknungseigenschaften der erhaltenen Form B, da nach der kurzen Trocknungsdauer von 3,5 h bei 40 °C die flüchtigen Anteile bereits entfernt waren.

### Beispiel 4: Herstellung der kristallinen Verbindung enthaltend Bazedoxifen und Milchsäure (Form C)

100 mg Bazedoxifen L-Laktat Form B werden in 3,5 ml Isopropanol bei 65 °C gelöst. Die klare Lösung wird innerhalb einer Stunde auf 50 °C und dann innerhalb zwei Stunden auf 10 °C abgekühlt. Es wird über Nacht gerührt und eine Suspension erhalten. Der Feststoff wird abfiltriert und über Nacht bei Raumtemperatur getrocknet. Das Röntgenpulverdiagramm ist in Figur 3 (Form C) angegeben. Es zeigt die in Tabelle 4 angegebenen Reflexe.

**Tabelle 4: Reflexe der Form C in °2θ und d-Werten**

| Pos. [°2θ.] | d-Werte [Å] | Qualitative Intensität |
|---|---|---|
| 7.4 | 12.0 | w |
| 7.7 | 11.5 | vw |
| 9.4 | 9.4 | w |
| 10.0 | 8.8 | m |
| 12.8 | 6.9 | s |
| 13.0 | 6.8 | m |
| 13.2 | 6.7 | w |
| 13.5 | 6.5 | m |
| 14.0 | 6.3 | m |
| 14.6 | 6.1 | w |
| 14.9 | 5.94 | m |
| 15.5 | 5.74 | m |
| 15.8 | 5.61 | VW |
| 16.5 | 5.36 | VW |
| 17.2 | 5.16 | w |
| 17.4 | 5.10 | s |
| 18.8 | 4.71 | s |
| 19.0 | 4.67 | m |
| 19.8 | 4.49 | w |
| 20.2 | 4.40 | vs |
| 21.0 | 4.23 | vs |
| 21.8 | 4.07 | m |
| 23.0 | 3.86 | m |
| 23.3 | 3.81 | m |
| 24.1 | 3.68 | m |
| 25.9 | 3.44 | w |
| 28.0 | 3.18 | w |
| 28.8 | 3.09 | VW |
| 29.4 | 3.03 | w |
| 29.5 | 3.02 | w |

### Beispiel 5: Herstellung der kristallinen Verbindung enthaltend Bazedoxifen und Milchsäure (Form C)

Die gemäss Beispiel 4 erhaltenen Kristalle werden in Methylethylketon suspendiert, die erhaltene Suspension wird bei Raumtemperatur für 40 Stunden gerührt und der Feststoff abfiltriert. Das Röntgenpulverdiagramm des Feststoffes entspricht dem der Form C. Eine thermogravimetrische Analyse die mit IR-Spektroskopie gekoppelt ist zeigt einen Gewichtsverlust des Produktes von 0,3%, wenn es mit 10 K/min auf 150 °C erhitzt wird. Dies bestätigt die sehr guten Trocknungseigenschaften der erhaltenen Form C.

## Patentansprüche

1. Kristalline Verbindung von 1-[4-(2-azepan-1-yl-ethoxy)-benzyl] -2-(4-hydroxyphenyl)-3-methyl-1H-indol-5-ol (INN: Bazedoxifene) der Formel 1 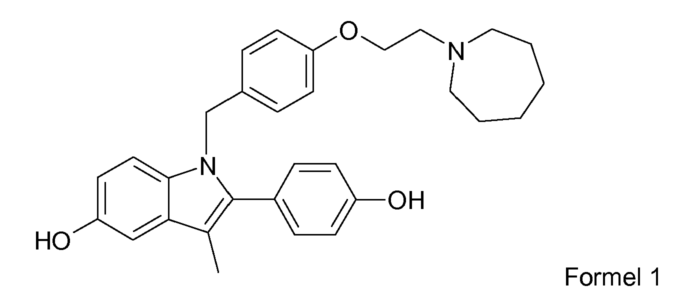 mit Milchsäure oder ein Hydrat hiervon, wobei das molare Verhältnis der Verbindung der Formel 1 zu Milchsäure 1:0,9 bis 1:1,1 ist.

2. Die kristalline Verbindung gemäss Anspruch 1, **dadurch gekennzeichnet, dass** die Verbindung ein Röntgenpulverdiffraktoramm (XRPD) mit wenigstens einem Reflex (ausgedrückt als 20 ± 0,2° 20 (Cu_{Ka} Strahlung)) bei 12.8°, 13.6°, 17.4°, 20.1° und 21.2° aufweist, die nachfolgend als Form A bezeichnet wird.

3. Die kristalline Verbindung gemäss Anspruch 2, **dadurch gekennzeichnet, dass** sie wenigstens einen Reflex 20 bei 10.0°, 12.8°, 13.6°, 15.5°, 17.4°, 19.0°, 20.1°, 21.2° und 24.3° aufweist.

4. Die kristalline Verbindung gemäss Anspruch 1, **dadurch gekennzeichnet, dass** die Verbindung ein Röntgenpulverdiffraktoramm (XRPD) mit wenigstens einem Reflex (ausgedrückt als 20 ± 0,2° 20 (Cu_{Ka} Strahlung)) bei 12.4°, 15.9°, 18.5° und 20.4° aufweist, die nachfolgend als Form B bezeichnet wird.

5. Die kristalline Verbindung gemäss Anspruch 4, **dadurch gekennzeichnet, dass** sie wenigstens einen Reflex 20 bei 9.4°, 12.4°, 15.2°, 15.9°, 18.5°, 18.7°, 20.4°, 21.4° und 29.5° aufweist.

6. Die kristalline Verbindung gemäss Anspruch 1, **dadurch gekennzeichnet, dass** die Verbindung ein Röntgenpulverdiffraktoramm (XRPD) mit wenigstens einem Reflex (ausgedrückt als 20 ± 0,2° 20 (Cu_{Kα} Strahlung)) bei 21.0°, 23.3° und 24.1° aufweist, die nachfolgend als Form C bezeichnet wird.

7. Die kristalline Verbindung gemäss Anspruch 6, **dadurch gekennzeichnet, dass** sie wenigstens einen Reflex 20 bei 10.0°, 12.8°, 15.5°, 17.4°, 18.8°, 21.0°, 23.3° und 24.1° aufweist.

8. Die kristalline Verbindung gemäss wenigstens einem der Patentansprüche 1 bis 7 zur Anwendung in der Behandlung von Osteoporose, insbesondere von postmenopausaler Osteoporose.

9. Ein Medikament enthaltend die kristalline Verbindung gemäss wenigstens einem der Patentansprüche 1 bis 7.

10. Die Verwendung der kristallinen Verbindung gemäss wenigstens einem der Patentansprüche 1 bis 7 zur Herstellung eines Medikamentes zur Behandlung von Osteoporose, insbesondere von postmenopausaler Osteoporose.

11. Verfahren zur Herstellung der Verbindung gemäss wenigstens einem der Ansprüche 1 bis 7 umfassend die Schritte:
a) Bereitstellung einer Lösung von 1-[4-(2-azepan-1-yl-ethoxy)-benzyl] -2-(4-hydroxyphenyl)-3-methyl-1H-indol-5-ol (INN: Bazedoxifene) der Formel 1 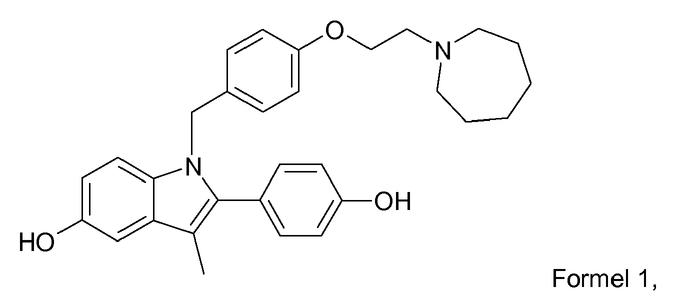
b) Zugabe von Milchsäure zu der Lösung des Schrittes a);
c) optionales Aufkonzentrieren der nach Schritt b) erhaltenen Zusammensetzung und/oder optionale Zugabe eines geeigneten Nichtlösungsmittels zur Erniedrigung der Löslichkeit der kristallinen Verbindung und
d) Abtrennung des erhaltenen Feststoffes.

12. Das Verfahren gemäss Anspruch 11, **dadurch gekennzeichnet, dass** das molare Verhältnis der gelösten Verbindung der Formel 1 in Schritt a) zu Milchsäure in Schritt b) 1:0,9 bis 1:1,1 ist.

13. Das Verfahren gemäss Anspruch 11 oder 12, **dadurch gekennzeichnet, dass** Milchsäure in Schritt b) in Substanz zu der Lösung des Schrittes a) gegeben wird oder die Säure des Schrittes b) als eine Lösung in einem geeigneten Lösungsmittel zu der Lösung des Schrittes a) gegeben wird.

14. Das Verfahren gemäss wenigstens einem der Ansprüche 12 bis 13, **dadurch gekennzeichnet, dass** das Lösungsmittel des Schrittes a) und/oder b) ein niedermolekulares und physiologisch verträgliches Lösungsmittel und/oder ein physiologisch verträglicher Alkohol ist.

15. Das Verfahren gemäss wenigstens einem der Patentansprüche 11 bis 14, **dadurch gekennzeichnet, dass** die Verbindung der Formel 1 in Schritt a) in einer Lösung enthaltend Ethanol, Ethylacetat und/oder Isopropanol vorgelegt wird.

16. Das Verfahren gemäss Patentanspruch 15, **dadurch gekennzeichnet, dass** in Schritt b) eine Lösung enthaltend Milchsäure und Ethanol, Ethylacetat und/oder Isopropanol als Lösungsmittel zugegeben wird.
